# FASCICULE DE BREVET EUROPEEN

(11) **EP 1 558 160 B1**
(45) Date de publication et mention de la délivrance du brevet: **05.04.2006**
(21) Numéro de dépôt: 03782531.2
(22) Date de dépôt: 07.11.2003
(51) Int. Cl.: A61B 17/80

(54) **ECROU DE SERRAGE POUR DISPOSITIF D OSTEOSYNTHESE**
SPANNMUTTER FÜR EINE OSTEOSYNTHESEVORRICHTUNG
CLAMPING NUT FOR OSTEOSYNTHESIS DEVICE

(30) Priorité: 08.11.2002 FR 0214038
(43) Date de publication de la demande: 03.08.2005
(73) Titulaire: SCIENT'X, 78284 Guyancourt (FR)
(72) Inventeur: CARLI, Olivier, CH-1207 Genève 6 (CH)
(74) Mandataire: Thibault, Jean-Marc
(86) Numéro de dépôt international: PCT/FR2003/003331
(87) Numéro de publication internationale: WO 2004/043276

(56) Documents cités:
- EP-A- 0 933 065
- EP-A- 0 997 107
- WO-A-98/41160
- DE-U- 29 522 089
- US-A- 5 147 363
- US-A- 5 151 103

## Description

La présente invention concerne le domaine technique de l'ostéosynthèse, notamment du rachis, et elle vise plus précisément un écrou de serrage utilisé dans les dispositifs d'ostéosynthèse pour le rachis.

L'objet de l'invention vise plus précisément un écrou de serrage conçu pour présenter une angulation relative par rapport à un appareillage d'ostéosynthèse au sens général.

Une des applications connues de l'objet de l'invention concerne les dispositifs conçus pour corriger et maintenir dans une position déterminée un ensemble de vertèbres. Selon cette application, un tel dispositif comporte un élément de liaison intervertébral, tel qu'une plaque ou tige, apte à être maintenu en position par des éléments d'ancrage osseux tels que des vis implantées dans les vertèbres. Chaque élément d'ancrage osseux est pourvu en tête, d'un axe fileté servant au montage de la plaque qui comporte des trous de passage. La plaque est emmanchée, par un trou de passage sur l'axe fileté dont une partie saillante, par rapport à la plaque, permet le vissage d'un écrou de serrage.

Compte tenu de l'anatomie des vertèbres, il apparaît nécessaire d'autoriser une angulation relative adaptative entre la vis d'ancrage et l'élément de liaison intervertébral. Pour autoriser une telle angulation relative adaptative, il est connu d'équiper les vis d'ancrage d'une articulation sphérique pour recevoir l'élément de liaison intervertébral. Une telle articulation sphérique est aménagée entre la vis d'ancrage et l'axe fileté pour permettre une orientation pluridirectionnelle de l'axe fileté recevant l'élément de liaison intervertébral.

Le document DE 295 22 089 U sur lequel est basé le préambule de la revendication 1 montre une telle vis.

Si une vis d'ancrage du type à articulation sphérique autorise une angulation relative adaptative entre ladite vis et l'élément de liaison intervertébral, il doit être considéré que la réalisation d'une articulation sphérique sur une vis d'ancrage contribue à la fragiliser en raison de l'intégration d'une interface mobile pouvant même être à l'origine d'une rupture de l'élément de liaison intervertébral. Par ailleurs, il a été constaté que même en présence d'une vis d'ancrage du type à articulation sphérique, l'axe fileté ne s'étend pas toujours perpendiculairement en saillie par rapport à l'élément de liaison intervertébral compte tenu de l'anatomie des vertèbres et de la zone d'implantation de la vis d'ancrage. Il s'ensuit que l'écrou destiné à être vissé sur l'axe fileté ne peut pas être serré convenablement, dans la mesure où l'élément de liaison intervertébral ne s'étend pas perpendiculairement à l'axe fileté, de sorte que l'élément de liaison intervertébral subit des contraintes inadaptées et ne peut pas être immobilisé complètement ou avec une force de serrage suffisante.

L'objet de la présente invention vise donc à remédier aux inconvénients énoncés ci-dessus en proposant une technique permettant de bloquer efficacement un élément de liaison intervertébral sur un appareillage d'ostéosynthèse, quelle que soit leur angulation relative.

L'objet de l'invention vise donc à proposer un écrou de serrage permettant de s'adapter à l'angulation relative entre l'axe fileté d'un appareillage d'ostéosynthèse et un élément de liaison intervertébral.

Pour atteindre un tel objectif, l'écrou de serrage comporte un corps annulaire et un alésage taraudé destiné à coopérer avec l'axe fileté, le corps annulaire étant pourvu de moyens d'entraînement en rotation et d'une face transversale d'appui. Selon l'invention, il comporte :
- une bague montée dans un logement du corps annulaire et pourvue intérieurement de l'alésage taraudé et formant extérieurement avec le corps annulaire une articulation sphérique,
- et des moyens de blocage en rotation relatif entre la bague et le corps annulaire.

Selon une caractéristique préférée de réalisation, la bague est pourvue extérieurement d'une rotule coopérant avec le logement de forme complémentaire pour former l'articulation sphérique.

Selon une autre caractéristique de l'invention, les moyens de blocage en rotation sont formés par deux tétons s'étendant de façon diamétralement opposée à partir de l'extérieur de la bague ou du corps annulaire, chaque téton étant logé à l'intérieur d'une cavité aménagée dans le corps annulaire ou dans la bague.

Selon une autre caractéristique de l'invention, les cavités et les tétons sont dimensionnés de manière à autoriser l'articulation sphérique entre le corps annulaire et la bague.

Selon une caractéristique préférée de réalisation, le corps annulaire est constitué de deux demi-corps annulaires emprisonnant la bague et positionnés entre eux à l'aide d'au moins un pion de centrage et fixés ensemble à l'aide de moyens d'assemblage.

Selon une autre caractéristique préférée de réalisation, les moyens d'entraînement en rotation sont constitués par des pans aménagés sur le corps annulaire qui présente une section hexagonale ou autres.

Un autre objet de l'invention est de proposer un dispositif d'ostéosynthèse comportant :
- au moins un appareillage d'ostéosynthèse présentant un axe fileté,
- un élément de liaison intervertébral muni d'un passage pour l'axe fileté,
- et au moins un écrou de serrage vissé sur l'axe fileté de sorte que sa face d'appui transversale se trouve en contact sur l'élément de liaison intervertébral.

Diverses autres caractéristiques ressortent de la description faite ci-dessous en référence aux dessins annexés qui montrent, à titre d'exemples non limitatifs, des formes de réalisation de l'objet de l'invention.
La **fig. 1** est une vue en perspective montrant un exemple de réalisation d'un écrou de serrage conforme à l'invention.
Les **fig. 2** et **3** sont des vues en coupe transversale prises respectivement selon les lignes II-II et III-III de la **fig.1.**
Les **fig. 4** et **5** sont des vues, respectivement en perspective et en élévation, d'un dispositif d'ostéosynthèse mettant en oeuvre un écrou de serrage conforme à l'invention.

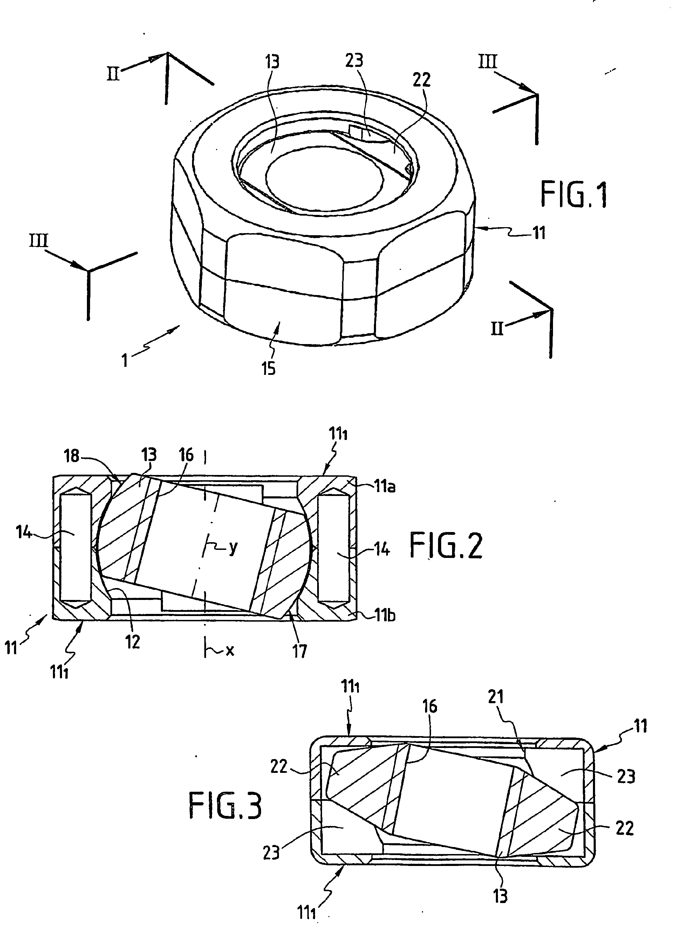

Les **fig. 1** à **3** illustrent un écrou de serrage **1** conforme à l'invention destiné à être utilisé dans un dispositif d'ostéosynthèse **2** pour le rachis.

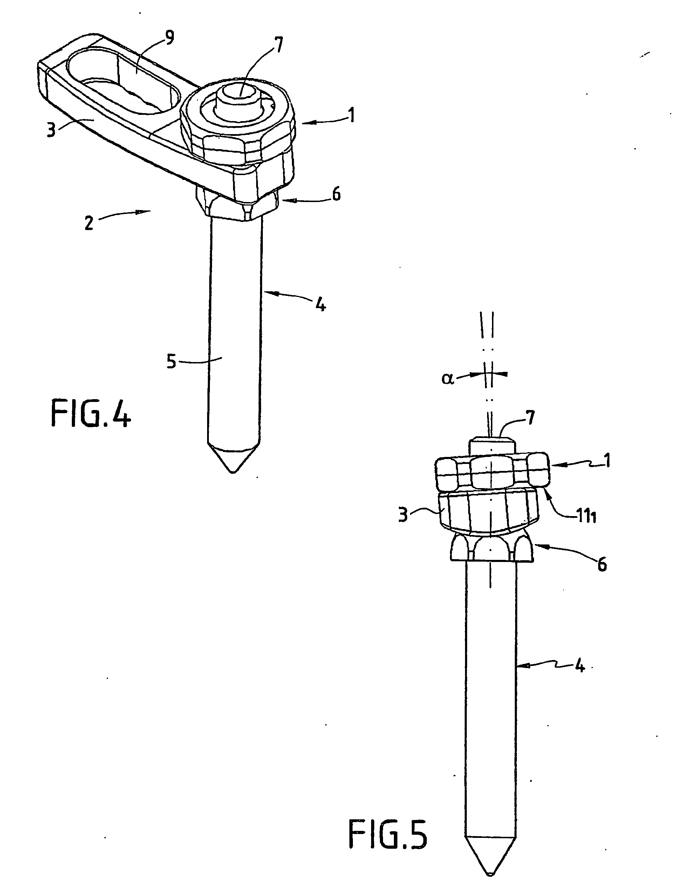

Dans l'exemple illustré aux **fig. 4** et **5,** le dispositif d'ostéosynthèse **2** comporte un élément de liaison intervertébral **3** tel qu'une plaque dans l'exemple illustré et au moins un appareillage d'ostéosynthèse **4** au sens général pouvant être réalisé de différentes manières connues. L'appareillage d'ostéosynthèse **4** constitue ainsi par exemple, un élément d'ancrage osseux se présentant sous la forme d'un crochet ou tel que représenté sur les dessins, d'une vis d'ancrage. Un tel appareillage d'ostéosynthèse **4** peut se présenter également sous la forme d'une plaque ou d'un élément intermédiaire de liaison.

Dans l'exemple illustré, l'appareillage d'ostéosynthèse **4** formant une vis d'ancrage osseux comporte, une partie filetée **5** et une tête **6** à partir de laquelle s'étend un axe fileté **7.** Dans l'exemple illustré, l'axe fileté **7** s'étend sensiblement dans le prolongement de la partie filetée **5**. De manière connue, l'élément de liaison intervertébral **3** est pourvu d'un ou d'une manière générale, de plusieurs trous ou passages **9.** Chaque trou **9** sert de passage à l'axe fileté **7** dont une partie fait saillie de l'élément de liaison intervertébral 3 pour permettre le vissage de l'écrou de serrage **1** conforme à l'invention.

Tel que cela ressort plus précisément des **fig. 1** à **3,** l'écrou de serrage **1** conforme à l'invention comporte un corps annulaire **11** délimitant intérieurement un logement **12** de réception pour une bague **13.** Le corps annulaire **11** possède ainsi un axe de révolution ou de symétrie **x** qui s'étend selon une direction sensiblement perpendiculaire par rapport aux faces transversales **11**_{**1**} du corps annulaire. Il est à noter qu'une face transversale **11**_{**1**} constitue une face d'appui destinée à être en contact avec l'élément de liaison intervertébral **3.**

Selon un exemple préféré de réalisation, le corps annulaire **11** est constitué de deux demi-corps annulaires **11a, 11b** emprisonnant la bague **13** et positionnés entre eux à l'aide d'au moins un et dans l'exemple illustré, de deux pions de centrage **14.** Les deux demi corps annulaires **11a, 11b** sont fixés ensemble à l'aide de tout type de moyens d'assemblage tels que par soudure par exemple.

Le corps annulaire **11** est pourvu de moyens d'entraînement en rotation **15.** Dans un exemple préféré de réalisation, les moyens d'entraînement en rotation **15** sont constitués par des pans aménagés sur le corps annulaire qui présentent ainsi par exemple, une section hexagonale. Bien entendu, les moyens d'entraînement en rotation **15** peuvent être réalisés de manière différente par exemple, par l'intermédiaire de trous de réception d'un instrument de manipulation.

Conformément à l'invention, la bague **13** est pourvue intérieurement d'un alésage taraudé **16** adapté pour coopérer avec l'axe fileté **7** d'un appareillage d'ostéosynthèse **4.** L'alésage taraudé 16 possède ainsi un axe de symétrie **y.**

Selon une autre caractéristique avantageuse de l'invention, la bague **13** et le corps annulaire **11** sont adaptés de manière à constituer ensemble une articulation sphérique **17** permettant de conférer à la bague **13** une angulation relative entre l'axe **y** de la bague et l'axe x du corps annulaire **11.** Dans l'exemple de réalisation illustré, la bague **13** est pourvue extérieurement d'une rotule **18** coopérant avec le logement **12** de forme complémentaire présenté par le corps annulaire **11.** Tel que cela ressort plus précisément de la **fig. 2,** la surface externe de la bague **13** présente ainsi un segment sphérique **18** apte à coopérer avec une surface sphérique complémentaire délimitant le logement **12** du corps annulaire.

Dans l'exemple illustré, la bague **13** est pourvue d'une rotule **18** présentant une surface sphérique convexe tandis que le logement **12** possède une surface en creux. Bien entendu, il peut être prévu d'intervertir les formes convexe et concave entre la bague **13** et le corps annulaire **11.**

Selon une autre caractéristique de l'invention, l'écrou de serrage **1** comporte des moyens **21** assurant un blocage en rotation relatif entre la bague **13** et le corps annulaire **11.** Les moyens **21** assurent le blocage de la bague **13** en rotation selon son axe y par rapport au corps annulaire **11.** Dans l'exemple de réalisation illustré, les moyens de blocage **21** sont formés par deux ergots ou tétons **22** s'étendant de façon diamétralement opposée à partir de l'extérieur de la bague **13.** Chaque téton **22** est logé à l'intérieur d'une cavité **23** aménagée dans le corps annulaire **11.** Bien entendu, les cavités **23** et les tétons **22** sont dimensionnés de manière à autoriser l'articulation sphérique entre le corps annulaire **11** et la bague **13.** Il est à noter qu'il peut être envisagé de réaliser les tétons **22** à partir du corps annulaire **11** de sorte que les cavités **23** sont aménagées dans la bague **13.**

L'écrou de serrage **1** selon l'invention possède l'avantage de présenter une angulation relative adaptative entre l'axe de la bague taraudée **13** et le corps annulaire **11** c'est-à-dire plus précisément une face transversale d'appui **11**_{**1**}**.** L'avantage de l'écrou de serrage **1** selon l'invention ressort clairement de la **fig. 5** montrant une plaque de liaison intervertébrale **3** inclinée d'un angle α par rapport à l'axe fileté **7.** Le vissage de la bague taraudée **13** sur l'axe fileté **7** conduit au contact de la face d'appui **11,** du corps annulaire **11** sur la plaque **3.** Compte tenu de la présence d'une articulation sphérique entre la bague taraudée **13** et le corps annulaire **11,** la face transversale **11**_{**1**} du corps annulaire vient en contact et en appui par toute sa surface sur la plaque **3.** La force de serrage de l'écrou est donc parfaitement maîtrisée et la plaque **3** ne subit pas de contraintes inadaptées.

L'invention n'est pas limitée aux exemples décrits et représentés car diverses modifications peuvent y être apportées sans sortir du cadre des revendications.

## Revendications

1. Ecrou de serrage apte à coopérer avec un axe fileté (7) d'un appareillage d'ostéosynthèse, l'écrou comportant un corps annulaire **(11)** et un alésage taraudé (16) destiné à coopérer avec l'axe fileté, le corps annulaire **(11)** étant pourvu de moyens d'entraînement en rotation **(15)** et d'une face transversale d'appui **(11**_{**1**}**), caractérisé en ce qu'**il comporte :
- une bague **(13)** montée dans un logement **(12)** du corps annulaire **(11)** et pourvue intérieurement de l'alésage taraudé **(16)** et formant extérieurement avec le corps annulaire **(11)** une articulation sphérique **(17),**
- et des moyens de blocage en rotation **(21)** relatif entre la bague **(13)** et le corps annulaire **(11).**

2. Ecrou de serrage selon la revendication 1, **caractérisé en ce que** la bague **(13)** est pourvue extérieurement d'une rotule **(18)** coopérant avec le logement **(12)** de forme complémentaire pour former l'articulation sphérique **(17).**

3. Ecrou de serrage selon la revendication 1, **caractérisé en ce que** les moyens de blocage en rotation **(21)** sont formés par deux tétons **(22)** s'étendant de façon diamétralement opposée à partir de l'extérieur de la bague **(13)** ou du corps annulaire **(11),** chaque téton **(22)** étant logé à l'intérieur d'une cavité **(23)** aménagée dans le corps annulaire (11) ou dans la bague **(13).**

4. Ecrou de serrage selon la revendication 1, **caractérisé en ce que** les cavités **(23)** et les tétons **(22)** sont dimensionnés de manière à autoriser l'articulation sphérique entre le corps annulaire **(11)** et la bague **(13).**

5. Ecrou de serrage selon l'une des revendications 1 à 4, **caractérisé en ce que** le corps annulaire **(11)** est constitué de deux demi-corps annulaires **(11a, 11b)** emprisonnant la bague **(13)** et positionnés entre eux à l'aide d'au moins un pion de centrage **(14)** et fixés ensemble à l'aide de moyens d'assemblage.

6. Ecrou de serrage selon la revendication 1, **caractérisé en ce que** les moyens d'entraînement en rotation **(15)** sont constitués par des pans aménagés sur le corps annulaire **(11)** qui présente une section hexagonale.

7. Dispositif d'ostéosynthèse du type comportant :
- au moins un appareillage d'ostéosynthèse **(4)** présentant un axe fileté **(7),**
- un élément de liaison intervertébral **(3)** muni d'un passage **(9)** pour l'axe fileté **(7),**
**caractérisé en ce qu'**il comporte au moins un écrou de serrage **(1)** conforme à l'une des revendications 1 à 6 vissé sur l'axe fileté **(7)** de sorte que sa face d'appui transversale **(11**_{**1**}**)** se trouve en contact sur l'élément de liaison intervertébral **(3).**

## Patentansprüche

1. Spannmutter, dazu geeignet, mit einer Gewindeachse (7) einer Osteosynthesevorrichtung zusammenzuwirken, wobei die Mutter einen ringförmigen Körper (11) und eine Gewindebohrung (16) umfaßt, die dazu vorgesehen ist, mit der Gewindeachse zusammenzuwirken, wobei der ringförmige Körper (11) mit Mitteln zum Versetzen in Rotation (15) und mit einer Querseite zum Halten (11₁) versehen ist,
**dadurch gekennzeichnet, daß** sie umfaßt:
- einen in einem Sitz (12) des ringförmigen Körpers (11) angebrachten und innen mit der Gewindebohrung (16) versehenen und außen mit dem ringförmigen Körper (11) eine sphärische Artikulation (17) bildenden Ring (13) und
- Mittel zum Blockieren der Rotation (21) relativ zwischen dem Ring (13) und dem ringförmigen Körper (11).

2. Spannmutter nach Anspruch 1, **dadurch gekennzeichnet, daß** der Ring (13) außen mit einem Kugelgelenk (18) versehen ist, das mit dem Sitz (12) von komplementärer Form zusammenwirkt, um ein sphärisches Gelenk (17) zu bilden.

3. Spannmutter nach Anspruch 1, **dadurch gekennzeichnet, daß** die Mittel zum Blockieren der Rotation (21) aus zwei Ansätzen (22) gebildet sind, die sich diametral gegenüberstehend von dem Äußeren des Rings (13) oder des ringförmigen Körpers (11) aus erstrecken, wobei jeder Ansatz (22) in einer Höhlung (23) sitzt, die in dem ringförmigen Körper (11) oder dem Ring (13) eingerichtet ist.

4. Spannmutter nach Anspruch 1, **dadurch gekennzeichnet, daß** die Höhlungen (23) und die Ansätze (22) so dimensioniert sind, daß die sphärische Artikulation zwischen dem ringförmigen Körper (11) und dem Ring (13) zugelassen wird.

5. Spannmutter nach einem der Ansprüche 1 bis 4, **dadurch gekennzeichnet, daß** der ringförmige Körper (11) aus zwei ringförmigen Halbkörpem (11a, 11 b) besteht, die den Ring (13) einschließen und zueinander mit Hilfe von wenigstens einem Zentrierbolzen (14) positioniert sind und mit Hilfe von Montiermitteln zusammengehalten sind.

6. Spannmutter nach Anspruch 1, **dadurch gekennzeichnet, daß** die Mittel zum Versetzen in Rotation (15) aus Stücken bestehen, die auf dem ringförmigen Körper (11) eingerichtet sind, der einen hexagonalen Querschnitt aufweist.

7. Vorrichtung zur Osteosynthese vom Typ, der umfaßt:
- wenigstens eine Osteosynthesevorrichtung (4), die eine Gewindeachse (7) aufweist,
- ein intervertebrales Element (3), das mit einem Durchgang (9) für die Gewindeachse (7) versehen ist,
**dadurch gekennzeichnet,**
**daß** sie wenigstens eine Spannmutter (1) nach einem der Ansprüche 1 bis 6 umfaßt, die auf die Gewindeachse (7) so geschraubt ist, daß ihre Querseite zum Halten (11₁) sich in Kontakt mit dem intervertebralen Verbindungselement (3) befindet.

## Claims

1. A locknut to fit onto a threaded rod (7) of an osteosynthesis appliance, where the nut has an annular body (11) and a threaded bore (16) designed to fit onto the threaded rod, and where the annular body (11) is equipped with the means by which it can be rotated (15) and a transverse bearing surface (11₁), **characterised in that** it includes:
- a ring (13) mounted in a recess (12) in the annular body (11), having on its inside the threaded bore (16), and forming on the outside, with the annular body (11), a spherical articulation (17),
- and resources for locking in relative rotation (21) between the ring (13) and the annular body (11).

2. A locknut according to claim 1, **characterised in that** the ring (13) is fitted on the outside with a swivel joint (18) fitting into the recess (12) of complementary shape, to form the spherical articulation (17).

3. A locknut according to claim 1, **characterised in that** the resources for locking in rotation (21) are formed by two nipples (22) located in a diametrically opposed manner from the outside of the ring (13) or from the annular body (11), each nipple (22) being accommodated inside a cavity (23) created in the annular body (11) or in the ring (13).

4. A locknut according to claim 1, **characterised in that** the cavities (23) and the nipples (22) are dimensioned so as to allow the spherical articulation between the annular body (11) and the ring (13).

5. A locknut according to one of claims 1 to 4, **characterised in that** the annular body (11) is composed of two annular half bodies (11a, 11b) trapping the ring (13), and positioned in relation to each other by means of at least one centring pin (14) and fixed together by means of assembly resources.

6. A locknut according to claim 1, **characterised in that** the means by which it can be rotated (15) are composed of flats created on the annular body (11), which is of hexagonal section.

7. An osteosynthesis device of the type that has:
- at least one osteosynthesis appliance (4) with a threaded rod (7),
- an intervertebral connecting element (3) in which there is a passage (9) for the threaded rod (7),
**characterised in that** it has at least one locknut (1) in accordance with any one of claims 1 to 6, screwed onto the threaded rod (7) so that its transverse bearing surface (11₁) is in contact with the intervertebral connecting element (3).
